# EUROPEAN PATENT APPLICATION

(11) **EP 4 840 492 A1**
(43) Date of publication of application: **18.11.2026**
(21) Application number: 25176398.3
(22) Date of filing: 14.05.2025
(51) Int. Cl.: A61M 15/08

(54) **METHOD FOR ASSESSING THE ORIENTATION OF A DRUG DISPENSER AS WELL AS DRUG DISPENSER AND ADD-ON TO CONDUCT THIS METHOD**

(71) Applicant: Aptar Radolfzell GmbH, 78315 Radolfzell (DE)
(72) Inventor: Walter, Matthieu, 78450 Villepreux (FR); Kohnle, Jörg, 78056 Villingen-Schwenningen (DE); Nocua, Ronald, 92320 Chatillon (FR); Neves de Oliviera, Mickael, 93270 Sevran (FR)
(74) Representative: Witte, Weller und Partner Patentanwälte mbB Stuttgart

(57) **Abstract**

The invention relates to a method and device for assessing the spatial orientation of a nasal drug dispenser during application. A reference orientation of the drug dispenser is first captured on the user's face, and at least one reference parameter is derived and stored. The current orientation of the drug dispenser is then evaluated based on this parameter, preferably during movement toward the nostril or during drug delivery. The evaluation system may classify the current orientation as proper or improper based on angular deviations, vector projections, or by comparison with a predefined optimal orientation. Optionally, a virtual cone may define an acceptable range around the optimal orientation. The invention further relates to a nasal drug dispenser and an add-on unit equipped with sensors and logic for capturing, evaluating, and optionally displaying the drug dispenser's orientation. Feedback to the user may be provided visually, acoustically, or haptically, and orientation assessment may be used to allow or inhibit drug release. The system may include a pivotable display, a force or actuation sensor, and use quaternions for real-time orientation tracking.

## Description

### FIELD OF THE INVENTION AND PRIOR ART

The present invention relates to devices and methods for the nasal delivery of pharmaceutical substances, and in particular to the assessment of the orientation of a nasal drug dispenser during application. A correct orientation of the drug dispenser relative to the user's anatomy is crucial for ensuring targeted and effective deposition of active agents, especially when aiming at the olfactory region.

Nasal drug delivery devices are well known and typically comprise a reservoir containing one or more doses of a fluid drug, a distribution head with a nozzle, and a mechanism for dispensing a metered dose, often in the form of a spray.

However, the efficacy of nasal delivery, even in spray form, can be significantly impaired when the device is misaligned in the nostril. This occurs frequently due to variations in user anatomy and the handling of the drug dispenser during application. Misalignment may result in reduced deposition in the olfactory region, which is particularly critical for therapies targeting the central nervous system.

Several approaches have been proposed in the prior art to guide the user towards a correct orientation or to automatically adjust the operation of the device based on orientation sensing. For instance, WO 2021/014076 A1 describes a nasal delivery assembly including an electronic module and a guidance system with orientation sensors both in the drug dispenser and in a separate head-mounted device, such as an earpiece, which cooperate to determine whether the drug dispenser is correctly positioned. The device may include automatic actuation features that trigger only when the optimal position is detected.

WO 2021/014078 A1 discloses a nasal delivery device equipped with a camera and indicator system. The camera determines the tilt angles of the drug dispenser relative to the user's facial axis by detecting specific facial landmarks. The drug dispenser can then be actuated automatically once optimal orientation is achieved. Visual and tactile indicators assist the user in reaching the target position.

WO 2019/155150 A1 teaches a nasal drug dispenser system that communicates with a remote mobile device (e.g., a smartphone), using accelerometers and gyroscopes to evaluate spatial orientation in real time. The system provides feedback to help the user align the device during actuation.

WO 2018/109409 A1 proposes a mechanical guidance solution based on a positioning structure with multiple facial contact points, designed to ensure consistent insertion angles.

Despite these advancements, there remains a need for systems that reliably assess drug dispenser orientation in real time, without relying on external hardware or complex facial tracking, and that can flexibly adapt to various application conditions, including differences in nostril anatomy.

### OBJECT OF THE INVENTION

The object of the invention is to enable reliable assessment of the orientation of a nasal drug dispenser during application in order to improve the precision and consistency of drug delivery.

To achieve this object, the invention provides a method for assessing the orientation of a nasal drug dispenser during application, wherein a reference orientation of the drug dispenser on the user's face is first captured, at least one reference parameter is derived from this orientation and stored, and subsequently, the current orientation of the drug dispenser is assessed based on this stored parameter, preferably during movement toward a nostril or during drug administration.

The invention relates to a method that enables a nasal drug dispenser to assess its own orientation during use. The core of this approach is the idea that the drug dispenser initially records how it is positioned on the user's face and this serves as a reference. From this starting position, one or more parameters are determined to describe the orientation of the device in space. These values are stored and used as a benchmark for further evaluation.

Once this reference has been set, the drug dispenser can monitor its current orientation and compare it with the previously recorded data. This comparison may take place, for example, while the device is being guided toward the nostril or during drug delivery. In this way, it becomes possible to check whether the drug dispenser is held in the intended position-or whether it has deviated from the optimal angle.

The process is designed to be user-friendly. There is no need for external equipment or fixed positioning aids. Instead, the device adapts to how it is naturally held and uses that position as its point of reference. By comparing subsequent movements to this initial state, the system can provide feedback or take action only when the conditions for proper drug delivery are met.

This approach offers a simple and reliable way to improve consistency in nasal applications, regardless of individual anatomy or posture.

It may be provided that the evaluation of the current orientation includes an assessment of how well the drug dispenser is aligned during drug delivery. In particular, the system may classify the orientation as either proper or improper.

This means that the drug dispenser not only compares its current position to the reference, but also interprets the result in a meaningful way, for example, by deciding whether the deviation remains within a tolerable range or whether it has become too large. The evaluation may rely on angular differences, vector projections, or other spatial parameters.

By translating raw orientation data into a simple binary output such as "proper" or "improper," the system can provide immediate feedback to the user or control other functions of the device, such as enabling or preventing drug release. This supports intuitive handling and reduces the risk of incorrect application, especially in situations where precise targeting is critical. Alternatively, the feedback to the user may also show more variants with acoustic or visual feedback also indicating how improper an improper orientation is.

It may be further provided that the classification or assessment of the orientation is based on whether the current position of the drug dispenser falls within a predefined acceptance range derived from the reference.

This approach allows the system to work with clearly defined thresholds, such as maximum allowed tilt angles or deviations from an optimal vector. These acceptance criteria can be tailored to the specific anatomy or specific drug requirements, ensuring, for example, that a certain spray cone reaches the intended area in the nasal cavity.

The classification helps distinguish between minor deviations that do not affect therapeutic outcome and more significant misalignments that may require correction. It also allows for visual or acoustic feedback that guides the user toward proper alignment.

It may be provided that the reference parameter includes a deviation angle, referred to as ψ, which describes the horizontal rotational component of the drug dispenser's longitudinal axis relative to a vertically defined axis in an Earth-fixed coordinate system. In other words, ψ indicates how the drug dispenser is oriented around the user's vertical axis in absolute terms, quite similar to a compass angle.

For example, when the user is facing north and the drug dispenser points straight forward, the angle ψ may be 0°. If the user turns west and again holds the drug dispenser straight forward, ψ would change to 90°, assuming an Earth-referenced coordinate frame.

Such an angle-based parameter allows the method to reliably evaluate orientation in real-world coordinates, even under changing user positions. This can help ensure consistent application quality, especially in systems where directional targeting within the nasal cavity plays a role.

It may be provided that the reference orientation is captured in connection with the triggering of an actuation element. This ensures that the orientation is recorded at a defined moment during device use, typically just before or during application.

Primarily, two different capture modes may be supported:
In a first variant, the actuation event itself directly triggers the capture of the reference orientation. This approach is straightforward and ensures that orientation data is always linked to an intentional user interaction.

In a second variant, after triggering the actuation element, the system waits until the device is in a substantially motionless state after actuation, and then automatically captures the orientation. This allows more stable and reliable data acquisition by reducing the impact of user-induced motion during triggering of the actuation element.

In a further alternative implementation, which deviates from the variants described above, the reference orientation may be captured solely based on motion characteristics, for example when the drug dispenser is detected to be substantially motionless, as determined by acceleration sensors, even without actuation. This allows reference acquisition without any mechanical trigger, making the system more autonomous and adaptive to various usage contexts.

It may be provided that the current orientation of the drug dispenser is evaluated in terms of a spatial vector, namely by the direction of its longitudinal axis. This vector may be projected onto two orthogonal planes, such as the sagittal plane (ZY) and the frontal plane (XZ), in order to assess angular alignment in both relevant directions.

Preferably, the system determines separately for each plane whether the corresponding projected angle lies within a predefined acceptance region. Only if the alignment is deemed acceptable in both planes is the overall orientation considered proper.

This dual-plane approach enables a simple and robust evaluation of orientation. It ensures that the device is not only tilted correctly in one direction (e.g. upward/downward), but also aligned laterally (e.g. toward the nasal cavity rather than the cheek). By requiring acceptance in both projections, the method reduces the likelihood of misalignment due to partial or one-sided deviations.

However, there are other possible modes of assessment:
It may be provided that the assessment of the drug dispenser's orientation includes a direct comparison between the current orientation and an optimal application orientation. Both orientations may be represented as spatial vectors.

Preferably, the evaluation is based on the angular deviation, i.e. the delta angle, between the current vector and the target vector. This angle provides a quantitative measure of how far the actual alignment deviates from the intended direction.

A threshold value may be defined, such that if the delta angle exceeds this limit, the current orientation is classified as improper. This allows the system to make a clear and reproducible decision about the correctness of the device alignment during use. By basing the evaluation on a single scalar angle derived from vector comparison, the method simplifies real-time analysis while maintaining geometric accuracy.

It may alternatively be provided that the optimal application orientation defines a virtual cone within which the current orientation must remain in order to be classified as proper. This cone represents a tolerance region around the target direction. The apex of the cone typically corresponds to the origin point of the spray or delivery axis, and the cone opens toward the intended direction of application.

The system checks whether the current orientation vector is located in the acceptance cone. If this is not the case, the orientation is deemed improper.

The cone can have various different geometrical shapes for the base. Also, complex shapes are possible to ensure very specific nasal regions for the application of the drug. A circular base results in a rotationally symmetric tolerance region, while a polygonal or elliptical base may be used to define more complex tolerances, for example, allowing more deviation in one direction than in another. This provides flexibility to adapt the acceptance region to anatomical or functional requirements of the specific application.

It may be provided that the method is designed to assess the drug dispenser's orientation separately for applications into the left and right nostril. In this context, the system determines into which nostril the drug is currently being administered, based on how the drug dispenser is held during use.

Preferably, this nostril-specific determination is achieved by comparing the current orientation vector with predefined optimal orientation vectors for the left and right nostrils, respectively. The nostril whose reference orientation best matches the current position can then be identified.

Alternatively, the determination may rely on the tilt direction of the drug dispenser with respect to a vertical axis in an Earth-fixed coordinate system, in particular with respect to the sagittal plane. For example, a tilt to the left may be interpreted as alignment toward the left nostril. This enables nostril classification without needing absolute reference vectors, and allows the method to function even in mobile or handheld use scenarios.

This nostril-specific detection can be useful both for orientation feedback and for triggering the appropriate control logic during drug delivery.

An alternative can be that a fixed sequence is given that the user has to follow.

It may be provided that the spatial orientation of the drug dispenser is represented and evaluated using quaternions. Quaternions offer a mathematically robust and efficient way to describe 3D rotations without the singularities and ambiguities associated with Euler angles.

In particular, the current orientation (B) may be expressed as a quaternion and compared with a stored reference orientation (A) and/or a predefined optimal application orientation (C), which are also stored as quaternions. The comparison may involve computing the angular difference or the rotation needed to align the current orientation with the reference or optimal direction.

Quaternions are especially well suited for embedded systems with limited processing capabilities. Compared to matrix-based or angle-based methods, quaternion operations require fewer computational steps and less memory. Therefore, they have been found to be optimal for a compact drug dispenser that relies on simple microcontrollers or resource-constrained signal processing hardware. Using quaternions allows smooth and continuous tracking of orientation changes, supports interpolation and averaging, and simplifies the implementation of rotation-based thresholds even in low-power devices.

In a further aspect of the invention, a drug dispenser for nasal application of pharmaceutical substances is provided. The drug dispenser comprises a drug reservoir and an outlet for delivering the drug into a nostril, as well as a mechanism for conveying the drug to the outlet, such as a pump, a spring-actuated delivery unit, or a pressure-based system with pre-pressurized fluid.

The drug dispenser is further equipped with an evaluation system designed to assess the spatial orientation of the device during use. This evaluation system is adapted to perform the method described above, including capturing a reference orientation and evaluating the current orientation with respect to it.

Preferably, the evaluation system is integrated directly into the drug dispenser housing and may include one or more sensors, such as accelerometers, gyroscopes, or magnetometers. These components may be mounted on a dedicated circuit board and connected to a microcontroller or signal processing unit.

The drug dispenser may comprise control logic that enables real-time processing of orientation data and optionally controls or blocks drug release in response to improper orientation. Feedback may be provided to the user through visual indicators, acoustic signals, or haptic feedback.

The evaluation unit may be powered by a battery integrated into the drug dispenser body. The overall construction may be designed as a single-use device or as a reusable unit with replaceable reservoirs. Preferably, the device is compact and lightweight and designed for intuitive, handheld operation without the need for external calibration tools.

By incorporating orientation evaluation directly into the physical structure of the drug dispenser, the system becomes self-sufficient and allows secure, user-independent monitoring of correct application.

The components for conducting the assessment method may be directly integrated into the drug dispenser, not being divisible by the end user. However, the components may also be added in an exchangeable and reusable add-on belonging to the drug dispenser.

Thus, in a further aspect of the invention, such an add-on unit for a nasal drug dispenser is provided. The add-on unit is designed to be mechanically and/or electronically coupled to a base drug dispenser and comprises the evaluation system for assessing the correct orientation of the coupled device during use.

This modular approach allows existing drug dispensers to be upgraded or retrofitted with orientation-sensing capabilities without requiring redesign of the dispensing mechanism itself. Preferably, the add-on unit is attached in the vicinity of the drug dispenser body, for example at the base, the actuator zone, or surrounding the reservoir.

The evaluation system integrated into the drug dispenser or the add-on unit may include motion and orientation sensors, such as accelerometers and gyroscopes, and a processing unit for executing the orientation method described above. The system may also comprise storage for reference parameters and logic for real-time classification of the current orientation.

The add-on unit or the drug dispenser itself may provide feedback to the user via LEDs, audible tones, vibration elements, or a small display. Audible tones may also include voice output to guide the user through the process. It may also communicate with the host drug dispenser to enable or inhibit drug delivery depending on whether a proper orientation has been detected.

Preferably, the add-on unit is powered by a battery and may include an activation switch or operate in a passive sensing mode. The physical design may follow a universal format for compatibility with different drug dispenser types or may be tailored to a specific product family.

The modular embodiment with an add-on offers flexibility for manufacturers and users, enabling orientation-aware application without requiring complete redevelopment of the dispensing hardware.

In a preferred development of the add-on unit, the system comprises at least one sensor configured to detect the actuation or application of the coupled drug dispenser. This sensor enables the system to associate orientation data with specific phases of use, such as the moment of drug release or the initiation of application. Preferably, the sensor is implemented as a pressure sensor or a force sensor. The sensor may be positioned such that it detects the force exerted on a carrier platform, a dispensing button, or another structurally defined interface of the drug dispenser when the user activates the device to discharge the drug.

The actuation detection may be used to trigger the capture of the reference orientation, initiate the assessment logic, or activate user feedback elements. In some embodiments, it may serve purely for documentation or compliance purposes-for example, by logging the time and orientation of each detected usage event.

The sensor system may be integrated seamlessly into the add-on housing and share processing and power resources with the orientation evaluation unit. Preferably, its use does not require any mechanical modification of the drug dispenser itself, allowing simple attachment and removal of the add-on.

In a further embodiment, the drug dispenser or the add-on unit may be equipped with an alignment sensor configured to detect the spatial orientation of the drug dispenser in real time. The alignment sensor may comprise at least one accelerometer, at least one gyroscope, and/or at least one magnetometer, preferably in the form of an integrated inertial measurement unit (IMU).

The accelerometer enables the detection of the direction of gravity and thus provides information about the tilt of the drug dispenser relative to the vertical. The gyroscope allows measurement of angular velocity, which can be used to track changes in orientation over time. A magnetometer, if present, may serve as a digital compass, allowing the system to determine absolute orientation with respect to the Earth's magnetic field.

Preferably, the sensor data is processed in the drug dispenser or add-on unit using sensor fusion algorithms that compensate for noise, drift, and transient motion. The result is a reliable estimation of the current spatial orientation of the drug dispenser, which can then be used as the basis for comparison with the reference orientation and/or optimal application orientation.

The alignment sensor may be positioned anywhere in or on the device where stable mechanical coupling to the drug dispenser body is ensured. For example, it may be integrated on a printed circuit board inside the housing or directly mounted in the add-on structure. Preferably, the sensor module is miniaturized and consumes little power, making it suitable for battery-operated portable devices.

In a preferred development, the drug dispenser or the add-on unit comprises a feedback system configured to output the result of the orientation assessment to the user. The purpose of this feedback is to inform the user in real time whether the current orientation of the device is within the acceptable range.

The feedback may be provided acoustically (e.g., via a beep or tone, or different tones indicating whether the orientation is improving or worsening), visually, or through haptic signals (e.g., vibration). Preferably, the feedback modality is chosen to be intuitive and perceivable during normal handling, without distracting from the application process.

In one preferred embodiment, the drug dispenser is equipped with a visual display element, such as an LCD, LED matrix, or indicator panel. The display may show a symbol, a color signal, or directional cues indicating the deviation between the current orientation and the target orientation.

For example, the display may include multiple direction indicators, such as arrows or sectors pointing in the direction in which the user must adjust the device to reach the correct orientation. These indicators may respond dynamically to the orientation data and guide the user step by step toward proper alignment.

The feedback system may also serve a gating function, allowing drug delivery only when a proper orientation is detected, or logging misaligned attempts for documentation purposes. Preferably, the feedback mechanism is designed to be energy-efficient and is integrated compactly into the device or add-on.

The drug dispenser or the add-on may be designed such that a display device is pivotally mounted on the base of the drug dispenser or the add-on unit. This enables the display to be folded into a storage position when not in use and pivoted into a visible position during application. Such a design helps protect the display element and reduce the size of the device during transport or storage while ensuring good visibility during use. Furthermore, the display or other visual elements can be brought into a position to be easily seen by the user by using said pivotable design.

Additionally or alternatively, the device may comprise a dedicated actuation element such as a button or touch sensor that allows the user to manually trigger the capture of the reference orientation. This provides an intuitive way to initiate calibration at a defined moment, particularly in situations where automatic detection is not desired.

In a further variant, the system may include logic for capturing the reference orientation based solely on motion characteristics, as described above. In this case, the system detects when the drug dispenser is in a sufficiently still state and triggers the reference capture without requiring any user interaction.

### BRIEF DESCRIPTION OF THE DRAWINGS

Further advantages and aspects of the invention will become apparent from the claims and from the following description of preferred embodiments of the invention, which are explained below with reference to the figures.
Fig. 1A shows a perspective view of a drug dispenser comprising a reservoir, an outlet, and an add-on unit for orientation evaluation.
Fig. 1B shows a side view of the drug dispenser of Fig. 1A.
Fig. 2 shows a perspective view on the drug dispenser and an add-on separated therefrom.
Fig. 3 shows a perspective view on the full drug dispenser with attached add-on.
Fig. 4 shows a schematic sectional view of the add-on unit with internal components, including an evaluation system and sensors.
Figs. 5A to 5C illustrate the positioning of the drug dispenser on the user's face and the capture of a reference orientation.
Figs. 6A to 6C show exemplary optimal application orientations of the drug dispenser relative to different spatial directions.
Figs. 7A to 7D illustrate improper orientations of the drug dispenser with respect to the target orientation.
Figs. 8A to 8D show further examples of misalignment outside the permissible range.
Figs. 9A to 9D depict orientations of the drug dispenser that are classified as proper.
Figs. 10A to 10C show different configurations of a virtual cone used for assessing the orientation, including circular, polygonal, and elliptical base shapes.
Fig. 11 illustrates a pair of cones representing the optimal application orientations for the left and right nostrils, respectively.

### DETAILED DESCRIPTION OF THE PREFERRED EMBODIMENTS

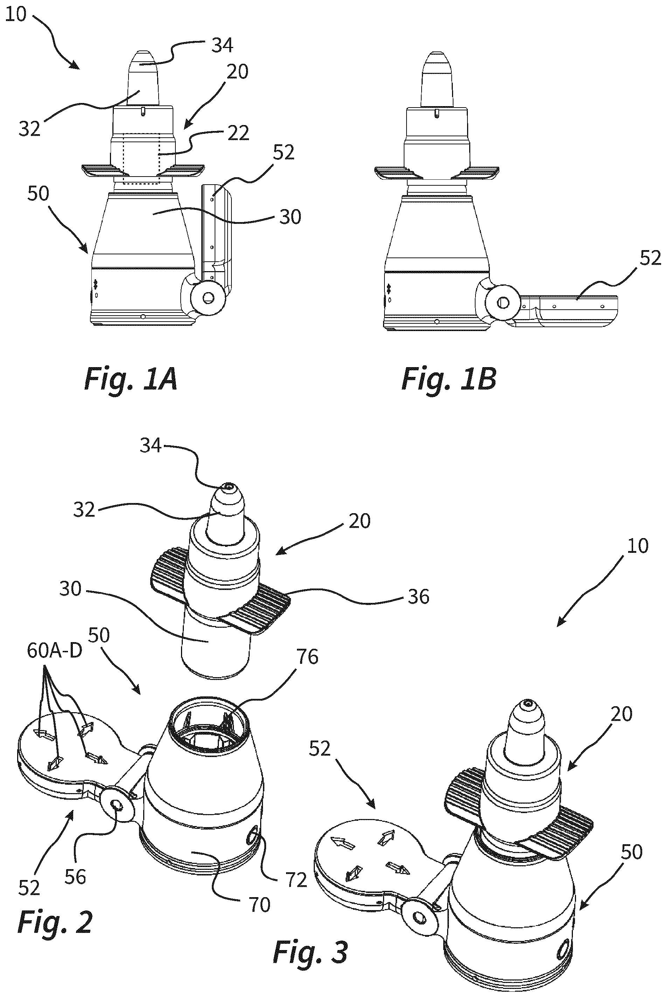

Fig. 1A shows a perspective view of a nasal drug dispenser 10 comprising a reservoir 30 for holding a pharmaceutical drug and an outlet 34 at the end of an applicator 32 for delivering the substance into a nostril of a user 100. The drug can primarily be in liquid form or in dry powder form. A delivery mechanism 22 such as a pump is provided to convey the drug from the reservoir 30 to the outlet 34. The drug dispenser 10 further includes an add-on unit 50, which is attached to the main body of the drug dispenser and houses an evaluation system for assessing the spatial orientation of the device during use. A pivotable section 52 is part of the add-on unit 50 and contains direction indicators 60A-60D. This pivotable section 52 is shown in Fig. 1A in a storage position.

Fig. 1B shows the same drug dispenser 10 with the pivotable section 52 in a use position.

Fig. 2 is a perspective view of the base drug dispenser 20 and its add-on 50, together forming the full and electronically assisted drug dispenser 10. The add-on 50 comprises a carrier platform 76 on which the reservoir 30 of the drug dispenser is to be placed. Furthermore, an actuation element 72 is provided on the side of base 70 of the add-on 50. As can also be seen in Fig. 2, the pivotable section 52 is swiveled to the use position using a hinge 56, such that direction indicators 60A-60D can be seen from above.

Fig. 3 shows the drug dispenser 20 and the add-on unit 50 coupled together and thus forming the full drug dispenser 10 with orientation assessment according to the invention. The reservoir 30 is placed on the carrier platform 76 and held there for example by friction. The drug dispenser 20 includes a finger rest 36 to trigger dispensing.

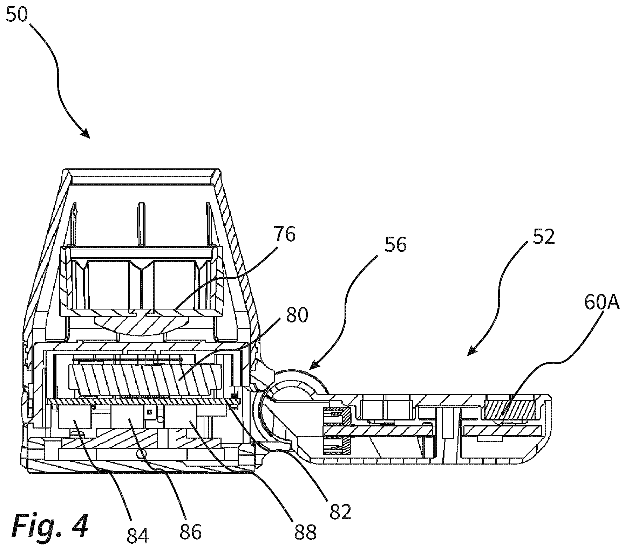

Fig. 4 is a schematic sectional view of the add-on 50, showing in particular a circuit board 82 placed inside the base 70 of the add-on 50. The add-on 50 includes a motion and orientation sensor 84, which may comprise an accelerometer, a gyroscope, and/or a magnetometer. A force sensor 80 is arranged to detect actuation events or user interaction with the drug dispenser, in particular to detect force applied to the carrier platform 76 during use. An electronic control module 86 processes sensor data and determines the current orientation of the drug dispenser 10.

By this, the assessment is conducted, leading to the output of signals using the direction indicators 60A-60D on the pivotable section 52 of the add-on 50.

Together, Figs. 1 to 4 illustrate a drug dispenser that is capable of monitoring its orientation in real time, referencing a user-defined starting position, and classifying the alignment during application. The add-on structure allows the orientation functionality to be modular and retrofittable, while enabling user-independent evaluation of proper usage.

In the following, the usage of this enhanced drug dispenser 10 is described.

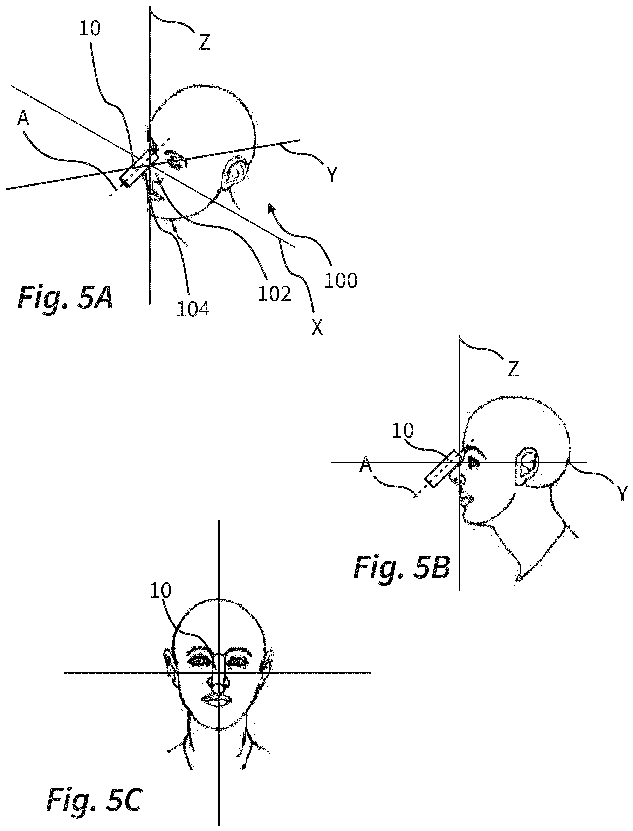

Figs. 5A to 5C illustrate a calibration process. For calibration, the drug dispenser 10 is placed on the face of a user 100, and a reference orientation A is defined. In these figures, the nose 102 and the nasal bridge 104 of the user are shown as the contact surface for calibration.

The drug dispenser 10 is placed in a position where it is resting against the nasal bridge 104 by the user, defining a reproducible alignment condition. This position may be used to capture the reference orientation A, which serves as a baseline for subsequent evaluations. The drug dispenser may be temporarily held still in this position and either manually or automatically detected by the system based on motion data, and the corresponding orientation vector is stored as a reference. Preferably, the user presses the actuation element 72 in order to start the calibration process.

The reference orientation A may be defined as the spatial direction of the drug dispenser's longitudinal axis at the moment of proper facial contact. This approach allows the reference to be individually adapted to the anatomy of the user, without requiring external fixtures or calibration tools.

After this calibration process, the face of the user has to remain still, not changing orientation, in order to allow subsequent assessment of the user's handling of the drug dispenser 10.

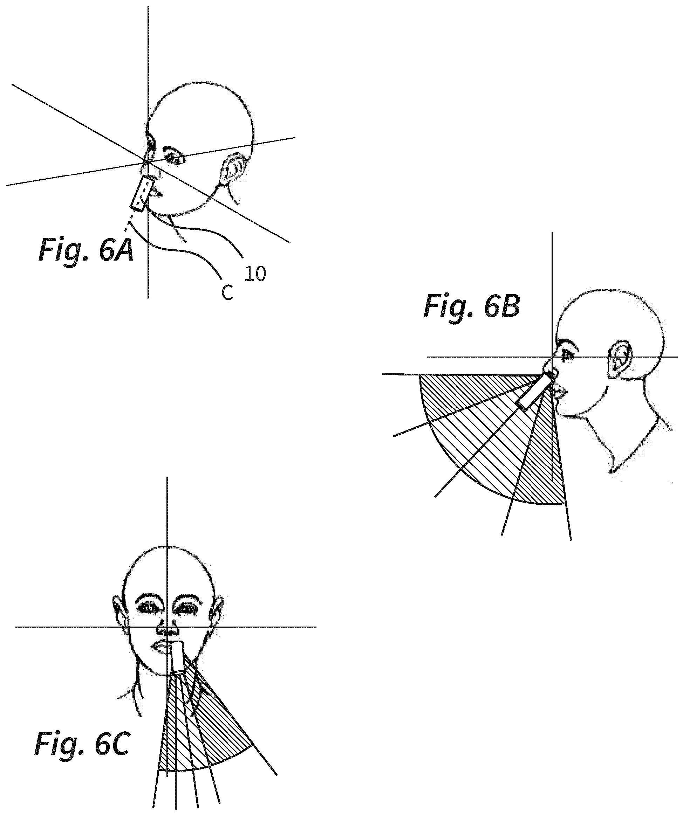

Figs. 6A to 6C show the ideal or optimal application orientations C of the drug dispenser 10 in three-dimensional space for the left nostril.

The drug dispenser 10 is shown in these figures in an exemplary position where proper drug delivery into the nasal cavity is expected to occur. The optimal orientation C is typically defined in advance-either during manufacturing, by user input, or through empirical optimization. The system is provided with stored data regarding this optimal orientation C relative to the reference orientation A.

The system is configured to assess whether the current orientation B during use corresponds with the optimal orientation C sufficiently. If not, a warning may be given, or delivery may be inhibited. By comparing the current state to this predefined ideal, the device ensures not only reproducibility but also medical effectiveness of the application.

In this and the following figures, the broadly hatched area represents a tolerance zone in which dispensing is regarded as proper, whereas the narrowly hatched area lies outside the tolerance zone and an application in such orientation is regarded as improper.

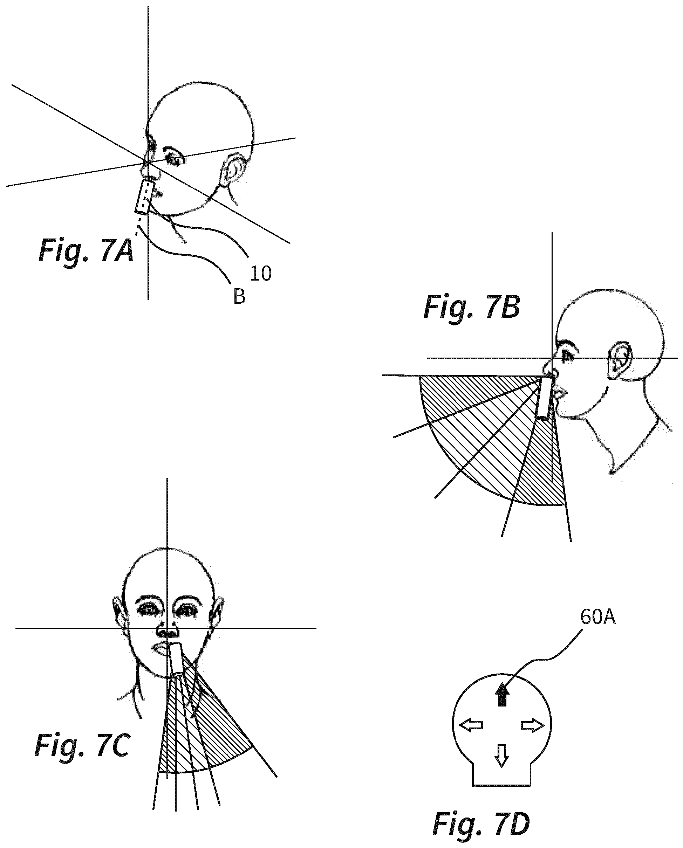

Figs. 7A to 7D illustrate an improper orientation of the drug dispenser 10 relative to the optimal orientation C. The drug dispenser 10 is held too upright. Thus, while the drug dispenser 10 is in a proper orientation regarding the frontal plane of Fig. 7C, it is in an improper orientation regarding the sagittal plane of Fig. 7B. As a result, the multiple direction indicators 60A-60D show this misalignment to the user by illuminating the upper arrow 60A, preferably in red.

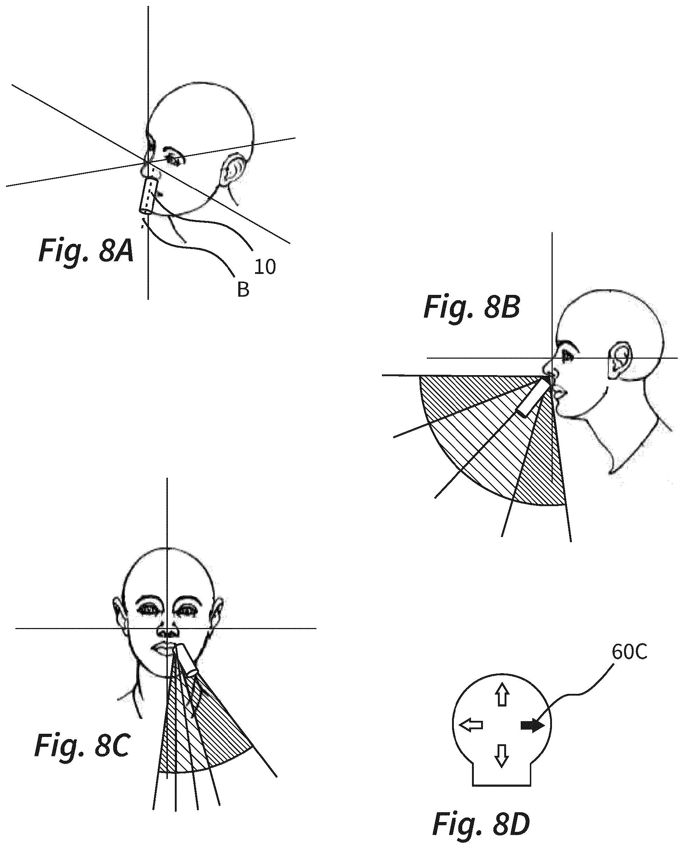

Figs. 8A to 8D show a further example of an improper orientation. In this situation, the drug dispenser 10 is in a proper orientation regarding the sagittal plane of Fig. 8B, but not in a proper orientation regarding the frontal plane of Fig. 8C. As a result, the multiple direction indicators 60A-60D show this misalignment to the user by illuminating the arrow 60C pointing to the right, preferably in red.

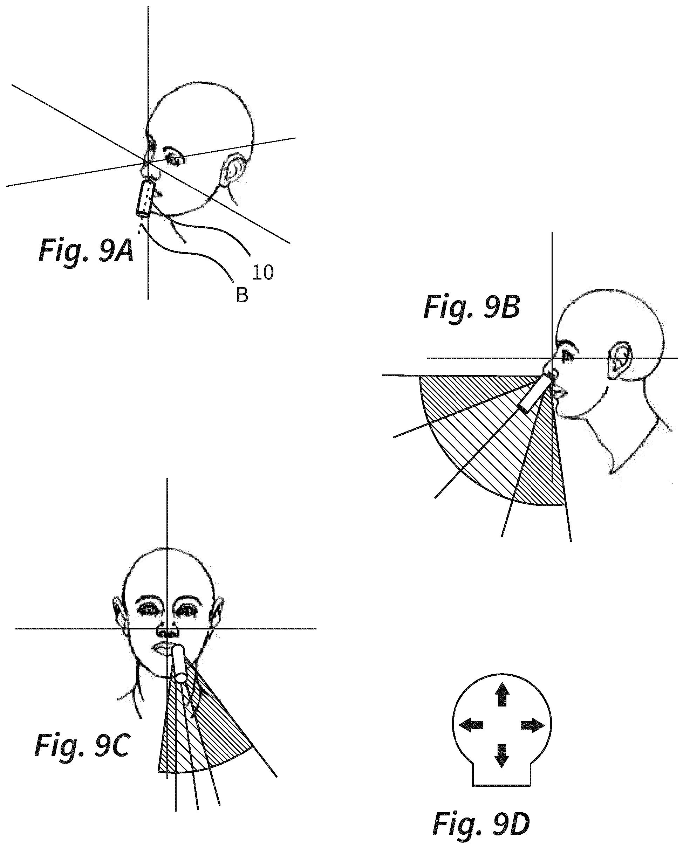

Figs. 9A to 9D depict the proper orientation the user has been guided to. The current orientation B in this state lies within the defined tolerance range regarding both planes, the sagittal plane of Fig. 9B and the frontal plane of Fig. 9C.

This success is shown to the user by illuminating all direction indicators 60A-60D simultaneously, preferably in green.

After having thus oriented the drug dispenser 10 in the correct direction, the user can discharge the drug by pressing down the finger rest 36 of the drug dispenser 20. This action is sensed by the force sensor 80. Preferably, the current orientation B is still determined during this application in order to verify that the user has not changed the orientation after having reached the proper one. The orientation during application can be stored for later review.

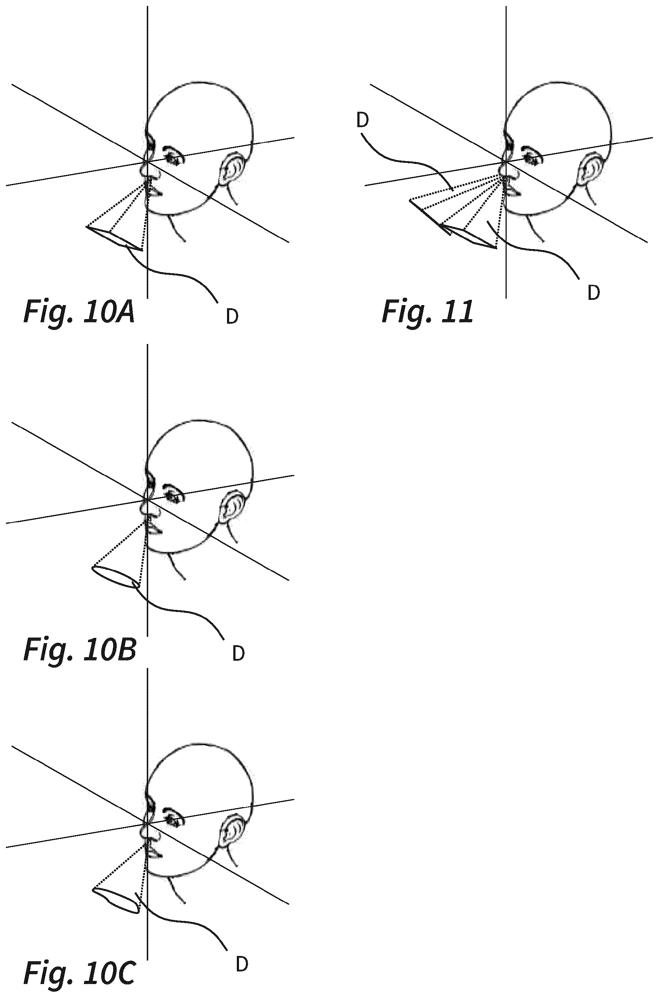

Figs. 10A to 10C illustrate another way of determining whether the current orientation is proper or improper. In this approach, a virtual cone D is defined by parameters stored in the memory of the drug dispenser or its add-on. The virtual cone D defines a three-dimensional acceptance region around or relative to the optimal orientation C. This cone is used to evaluate whether the current orientation B lies within a tolerable deviation from the ideal alignment.

In Fig. 10A, the base of the virtual cone D is rectangular, resulting in a pyramid-like tolerance volume. This is similar to the determination of proper and improper orientation based on the projections to the sagittal and the frontal planes.

In Fig. 10B, the base of the virtual cone D is circular, resulting in a rotationally symmetric tolerance volume. This allows for equal angular deviation in all radial directions around the axis defined by the optimal orientation C. Alternatively, an elliptic base shape may be used.

Fig. 10C shows a virtual cone D with a polygonal base, which may be used to introduce directionally selective tolerance, for example, to allow more deviation laterally than vertically, or to shape the acceptance region according to anatomical constraints or application safety margins.

The virtual cone D is defined such that the apex lies at the origin of the delivery axis, and the cone opens forward in the intended spray direction. The system classifies the orientation as improper if the current vector B falls outside this cone when crossing the apex.

Fig. 11 shows a configuration in which two virtual cones D1 and D2 are defined, corresponding to the optimal orientations for application into the left and right nostril, respectively. Each cone represents an acceptance region specific to one nostril, allowing the system to distinguish not only between proper and improper alignment, but also to determine which nostril is currently being targeted. However, the shape of the cones is usually identical and preferably there is a single parameter set defining the cones used for both nostrils.

This configuration supports nostril-specific guidance and feedback and may also serve to trigger internal logic, for example, dose tracking, left/right switching, or locking of the drug dispenser depending on orientation.

The invention is not limited to the embodiments shown in the drawings. Rather, modifications and variations are possible without departing from the scope of the invention as defined by the claims.

For example, the evaluation system may be implemented as a fully integrated solution within the drug dispenser or as a detachable module. The sensor arrangement, feedback mechanisms, and signal processing logic may be adapted to different device types, drug formulations, or therapeutic indications.

The methods described herein may be realized by hardware, software, or a combination thereof. In particular, the evaluation logic may be executed by a dedicated microcontroller embedded in the drug dispenser, by an external processing unit, or by a mobile application interfacing with the drug dispenser via wireless communication.

Unless otherwise stated, features described in connection with one embodiment may be combined with features of other embodiments in any technically meaningful way. The reference signs used in the claims do not limit the scope of the claims but serve merely to increase their clarity.

## Claims

1. Method for assessing the orientation of a drug dispenser (10, 20) when applying drugs into the nose (102) of a user (100), comprising the following features:
a. a reference orientation (A) of the drug dispenser (10, 20) on the face of the user (100) is captured, wherein at least one reference parameter is derived from said reference orientation (A) and is stored, and
b. after capturing the at least one reference parameter, an assessment is conducted regarding the current orientation (B) of the drug dispenser (10, 20) using the at least one reference parameter, wherein the assessment regarding the current orientation (B) is preferably conducted while the drug dispenser (10, 20) is being guided towards a nostril of the user (100) and/or during drug delivery.

2. Method according to claim 1 with the following additional feature:
a. the assessment of the current orientation (B) of the drug dispenser (10, 20) results in an evaluation of the quality of the orientation during drug delivery,
preferably with the following additional feature:
b. the assessment results in a classification of the orientation during drug delivery as either proper or improper.

3. Method according to any of the preceding claims with the following additional feature:
a. as reference orientation (A) on the face of the user the orientation of the drug dispenser (10, 20) on the user's nose (102) is used, particularly at the nose bridge (104).

4. Method according to any of the preceding claims with the following additional feature:
a. the at least one reference parameter includes a deviation angle describing a horizontal component of the drug dispenser's longitudinal axis about a vertically defined axis in an Earth-fixed coordinate system.

5. Method according to any of the preceding claims with the following additional feature:
a. the reference orientation parameter is captured in reaction to triggering of an actuation element (72) by the user (100),
preferably with one of the following additional features:
b. the reference orientation (A) is automatically captured when the drug dispenser (10, 20) is in a substantially motionless state following the triggering of an actuation element (72), or
c. the reference orientation (A) is captured when triggering of an actuation element (72).

6. Method according to any of the preceding claims with the following additional feature:
a. during the assessment, a vector representing the current orientation (B) is defined, and the respective angles of projections of this vector onto two planes (ZY, XZ) are assessed,
preferably with the following additional feature:
b. it is separately determined for each of the two planes (ZY, XZ) whether the respective projection lies within an acceptance region, and the result of the assessment is considered proper only if this is the case for both planes.

7. Method according to any of the preceding claims with the following additional feature:
a. during the assessment, a vector representing the current orientation (B) is compared to a vector representing an optimal application orientation (C),
preferably with the following additional feature:
a. the assessment whether the current orientation (B) during application is proper or improper is being judged based on a delta angle between the current orientation (B) and the optimal application orientation (C), wherein preferably a fixed maximum delta angle is defined above which the current orientation (B) is classified as improper.

8. Method according to claim 7 with the following additional feature:
a. a virtual cone (D) is defined by the optimal application orientation (C), wherein the current orientation (B) is classified as improper if it is not contained within the cone when crossing the cone apex,
preferably with the following additional feature:
b. the virtual cone (D) has a circle-shaped base, or
c. the virtual cone (D) has a polygonal-shaped base, or
d. the virtual cone (D) has an elliptic-shaped base.

9. Method according to any of the preceding claims with the following additional features:
a. the method is applied to assess the orientation of the drug dispenser (10, 20) during application into two nostrils of the user (100), and
b. the method comprises a determination into which nostril the drug is currently being applied,
preferably with the following additional features:
c. the determination of the nostril is based on a comparison of the current orientation (B) with an optimal application orientation (C) for the left and right nostril, respectively, or
d. the determination of the nostril is based on a tilt direction of the drug dispenser (10, 20) relative to a vertical axis in an Earth-fixed coordinate system.

10. Method according to any of the preceding claims with at least one of the following additional feature:
a. the assessment of the current orientation (B) is carried out using quaternions to represent and compute the spatial orientation of the drug dispenser (10, 20), preferably including a comparison of a quaternion representing the current orientation (B) with a quaternion representing the reference orientation (A) and/or the optimal application orientation (C).

11. Drug dispenser (10, 20) for nasal application of pharmaceutical drugs, comprising the following features:
a. the drug dispenser (10, 20) has a drug reservoir (30) and an outlet (34) for dispensing the drug into a nostril, and
b. the drug dispenser (10, 20) has a mechanism (22) for conveying drug to the outlet (34), and
c. the drug dispenser (10, 20) has an evaluation system for assessing a correct orientation of the drug dispenser,
**characterized by** the following feature:
d. the evaluation system is designed to conduct the method according to any of claims 1 to 10.

12. Add-on unit (50) for a drug dispenser (20) with the following features:
a. the add-on unit (50) is designed to couple to a drug dispenser (20) for nasal application of pharmaceutical drugs, and
b. the add-on unit (50) has an evaluation system for assessing a proper orientation of the drug dispenser,
**characterized by** the following feature:
c. the evaluation system is designed to conduct the method according to any of claims 1 to 10.

13. Add-on unit according to claim 12 with the following feature:
a. The add-on unit (50) has a sensor (80) for detecting the application operation of the coupled drug dispenser (20),
preferably with the following additional features:
b. the sensor (80) for detecting operation is designed as a pressure sensor or force sensor (80) and particularly integrated to detect the force acting on a carrier platform (76) when the drug dispenser is activated, and/or
c. the evaluation system is designed to detect the operation by analyzing the temporal progression of an applied force over time.

14. Drug dispenser (10) or add-on unit (50) according to any of claims 11 to 13 with the following additional features:
a. the drug dispenser (10) or the add-on unit (50) has an alignment sensor (84) by which the alignment of the drug dispenser (10) in space can be detected, wherein the alignment sensor (84) includes at least one accelerometer, at least one gyroscope, and/or at least one magnetometer, and
b. using the alignment sensor (84), the reference orientation (A) is captured, and the at least one reference parameter is derived from it.

15. Drug dispenser (10) or add-on unit (50) according to any of claims 11 to 13 with the following additional feature:
a. the drug dispenser (10) or add-on unit (50) comprises a feedback system which outputs the result of the assessment of the current orientation (B) of the drug dispenser (10), in particular by using an audio output or a visual output,
preferably with the following additional feature:
b. the drug dispenser (10) or add-on unit (50) has a display device (60) that shows the result of the assessment of the current orientation (B) of the drug dispenser (10), wherein the display (60) preferably has multiple direction indicators (60A-60D) that show the deviation of the current orientation (B) from a target orientation based on the reference parameter.

16. Drug dispenser (10) or add-on unit (50) according to any of claims 11 to 15 with the following additional features:
a. the display device (60) is pivotally mounted on a base of the drug dispenser (10) or add-on unit (50) and can be pivoted between a storage position and a use position, and/or
b. an actuation element (72) is provided to trigger the capture of the reference orientation (A) of the drug dispenser (10, 20) on the face of a user.
